# EUROPEAN PATENT SPECIFICATION

(11) **EP 4 358 816 B1**
(45) Date of publication and mention of the grant of the patent: **30.07.2025**
(21) Application number: 22734437.1
(22) Date of filing: 01.06.2022
(51) Int. Cl.: A61B 1/00, A61B 1/12, A61C 19/00

(54) **DENTAL POD**
DENTAL-POD
NACELLE DENTAIRE

(30) Priority: 22.06.2021 US 202117354652
(43) Date of publication of application: 01.05.2024
(73) Proprietor: DENTSPLY SIRONA Inc., York, PA 17401 (US)
(72) Inventor: BERNHARD, Nadine, 64625 Bensheim (DE); SATTLER, Joost, 64625 Bensheim (DE)
(74) Representative: Allen, Caroline Margaret
(86) International application number: PCT/US2022/031781
(87) International publication number: WO 2022/271423

(56) References cited:
- KR-A- 20170 111 585
- US-A1- 2017 100 225
- US-A1- 2018 333 232

## Description

### TECHNICAL FIELD

The disclosure relates generally to a dental pod and, more specifically, to a device and system for providing a localized access to features for dental handheld device use and maintenance.

### BACKGROUND

Dental handheld devices such as intraoral cameras allow clinicians to capture and display digital signals such as images from inside a patient's mouth. Between treatments, the intraoral camera is disinfected, wherein the clinician uses disinfection wipes to clean the camera. Data communication is done by a connection to an adjacent treatment unit. Color calibration and 3D calibration are executed manually with independent calibration kits. In effect, most steps require separate tools and features. For example, disinfection wipes and autoclaves may be required for the sterilization of handsets, and for color calibration, the clinician mounts a color calibration kit prior to performing manual color calibration steps based on visual instructions from a software.
Reference is made to the prior art document KR 20170111585 A. It discloses an apparatus for disinfecting a mouth scanner, the apparatus comprising: an insertion port into which a probe of the oral scanner is inserted.

### BRIEF SUMMARY

The aforementioned problems of the prior art can be overcome by the dental pod as defined in claim 1, and the method as defined in claim 14.
The illustrative embodiments provide a dental pod and a method of operating the dental pod. The dental pod provides a plurality of functions that are localized at the pod and shorten cleaning, setup, maintenance and other operations of and on a dental handheld device. An intraoral camera is disclosed as a dental handheld device for illustration. However other dental handheld device such as those configured to provide signals from a patient's dental cavity to a screen or computer for display can be conceived in light of the descriptions herein. Such dental handheld devices include, for example, dental caries detectors and apex locators.

In an aspect, the functions are performed automatically and include cleaning of the intraoral camera, charging of the intraoral camera, data communication of the intraoral camera via W-LAN (wireless local area network) to the pod and from there via LAN to a WLAN connected PC, intraoral camera calibration for color and 3D measurement, heating of the intraoral camera, fast cooling of the camera, configuring LED lights on the pod to display a charging status of a battery of the intraoral camera, direct operation at the pod via buttons. Further, the cleaning may be achieved in between each dental treatment of a patient. During this action, the intraoral camera charges independently. At any time, the camera may be calibrated automatically or with the pushing of a button. Data communication, through the upload of actual firmware software can be done at that docking station/pod, since it may be connected with a PC (personal computer) via LAN. Thus, a larger volume of data transmission is possible. The pod may act as a WLAN access point for other WLAN devices nearby (if the pod is equipped with such a component/provides this functionality). If an intraoral camera or the AC unit would use WLAN for communication then the pod could also be used, because it would provide the required access point to the practice LAN. Thus, a device and system for controlling an intraoral camera is disclosed. The device and system include a receiver for a distal end of a camera, the receiver includes nozzles for disinfection and cleaning as well as a heating/drying plate. A pump and ventilator in the pod are utilized to dispose disinfection liquids over the distal end of the intraoral camera (camera tip). The system further includes insertion slots for different cartridges and extensions for future needs to support several functions. A motor to turn the cartridges below the camera tip is also included to perform different functions. A positioning mechanism is used to move the camera into the right position, such as into the cartridge, for an individual cartridge in use. The system also includes a WLAN access point, an LAN slot, power plug to power the whole unit, and has touch and sound capabilities for device feedback and user input. One of the cartridges may include a disinfection liquid.

In an aspect herein, a dental pod is disclosed. The dental pod comprises: a receiver in a form of a cavity, adapted to receive a distal end of a dental handheld device; one or more nozzles disposed on a side of the receiver, the one or more nozzles configured to clean and disinfect the distal end of the dental handheld device; and a cartridge compartment disposed proximal to the receiver, the cartridge compartment including one or more insertion slots for one or more cartridges, the one or more cartridges configured to support operations of the handheld device.

In another aspect, the dental pod includes more than one combinations of the following features: (i) the dental handheld device is an intraoral camera, a dental caries detector or an apex locator, (ii) a motor turns the one or more cartridges to a predefined spatial position relative to the receiver to perform said operations of the dental handheld device, (iii) the pod is configured to alter a shape of the receiver to move the distal end of the dental handheld device into or out of an entrance of the one or more cartridges, this may be achieved, for example, via a linear actor e.g. a motor, a lead screw or a timing belt and a linear slide to move the device up and down. (iv) the one or more cartridges include at least one of a color calibration cartridge, a 3D calibration cartridge, a disinfection cartridge, a dental caries cartridge, for example for calibrating a dental caries detector, which may be achieved by a material combination that mimics certain caries situations for calibration, and an air-filter cartridge, (v) the dental pod includes a heater disposed along the side of the receiver or proximal to the receiver to heat at least the distal end of the dental handheld device after a cleaning operation, (vi) the one or more nozzles is operatively coupled to a pump for delivering fluids to the distal end of the dental handheld device, (vii) the fluids include at least one of fluids selected from the group consisting of spray water, spray air, a cleaning liquid and a disinfectant, (viii) the cartridge compartment is disposed below the receiver, (ix) the engine compartment is disposed below the cartridge compartment, (x) the pod is configured to communicate wirelessly with the dental handheld device, (xi) one or more insertion guides guide the insertion and retaining of the one or more cartridges in the cartridge compartment, (xii) an internal processor of the pod is configured to control said operations of the dental handheld device, (xiii) an external processor is configured to control said operations of the dental handheld device, (xiv) the receiver is shaped to allow manual movement of the distal end of the dental handheld device into or out of an entrance of the one or more cartridges.

In yet another aspect, a computer-assisted method of operating a dental pod is disclosed. The computer-assisted method of operating the dental pod, comprising the steps of: Automatically performing, by a processor, one or more operations of the dental handheld device based on a predefined function of the one or more cartridges, at least one of the one or more operations being selected from the group consisting of a color calibration operation, a 3D(three-dimensional) calibration operation, a cleaning operation, a disinfection operation, caries detection, and a spray air-filtering operation.

In an even further aspect, the computer-assisted method includes one or more of the following: (i) the automatically performing step includes a maintenance step of the dental handheld device, an automatic set-up step of the dental handheld device or an automatic cleaning step of the dental handheld device, (ii) operating a motor to turn at least one of the one or more cartridges to a predefined spatial position that is in alignment with the distal end of the dental handheld device in order to perform said one or more operations of the dental handheld device, (iii) verifying that a distal end of the dental handheld device has been received by the receiver and verifying that one or more cartridges are available in the cartridge compartment, the one or more cartridges being configured to support one or more operations of the dental handheld device, (iv) transferring data from the dental handheld device to a computer via a network of the pod and upload of actual firmware software and charging of the intraoral camera, (v) the dental handheld device is cleaned, heated and calibrated automatically, (vi) the dental handheld device is an intraoral camera, a dental caries detector or an apex locator.

### BRIEF DESCRIPTION OF THE SEVERAL VIEWS OF THE DRAWINGS

To easily identify the discussion of any particular element or act, the most significant digit or digits in a reference number refer to the figure number in which that element is first introduced.

Certain novel features believed characteristic of the invention are set forth in the appended claims. The invention itself, however, as well as a preferred mode of use, further objectives and advantages thereof, will best be understood by reference to the following detailed description of the illustrative embodiments when read in conjunction with the accompanying drawings, wherein:
FIG. 1A illustrates a perspective view of a pod in accordance with an illustrative embodiment.
FIG. 1B illustrates a perspective view of a pod in accordance with an illustrative embodiment.
FIG. 2 depicts a vertical cross section of a pod in accordance with an illustrative embodiment.
FIG. 3 illustrates a perspective view of a schematic of a pod in accordance with one illustrative embodiment.
FIG. 4 depicts a horizontal cross section of a pod in accordance with an illustrative embodiment.
FIG. 5 is a perspective view of a cartridge section of a pod and a motor according to one illustrative embodiment.
FIG. 6 illustrates a process in accordance with one embodiment.
FIG. 7 illustrates an aspect of the subject matter in accordance with one embodiment.
FIG. 8 depicts a block diagram of a computer system in which illustrative embodiments may be implemented.

### DETAILED DESCRIPTION

The illustrative embodiments described herein are directed to a pod having cartridges for a plurality of dental use-cases and configured to keep an intraoral camera ready for operation.

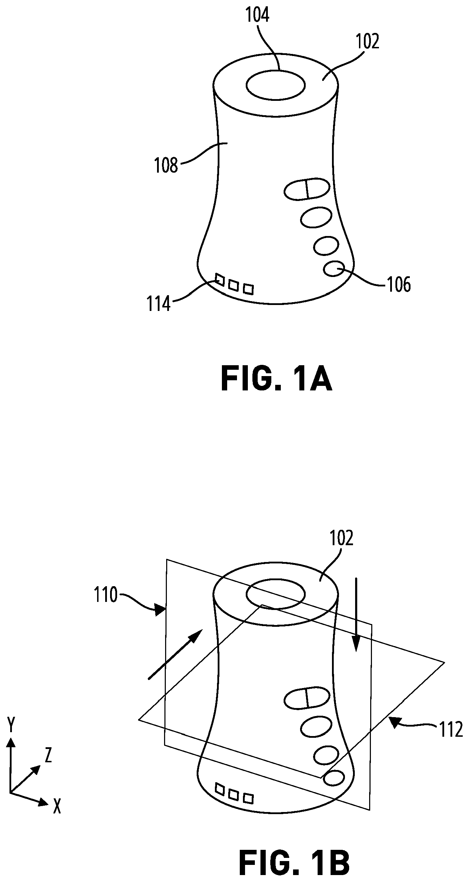

Reference will now be made to FIG. 1A, which shows a perspective view of a pod 102. The pod 102 comprises a housing 108, that encloses a receiver 104, the receiver 104 being configured as a cavity to receive and/or store at least a distal end of an intraoral camera and other dental handheld devices. The housing also includes one or more buttons 106 for operating the pod and a plurality of other internal components as discussed hereinafter, and power/data lines 114 for supplying power and/or data input/output to the pod 102. In an illustrative embodiment, the intraoral camera 216 may be powered by an internal battery and may be in wireless communication with a computer.

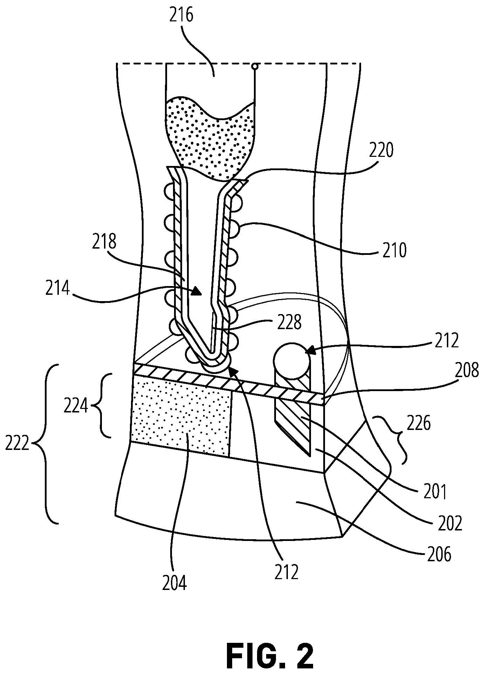

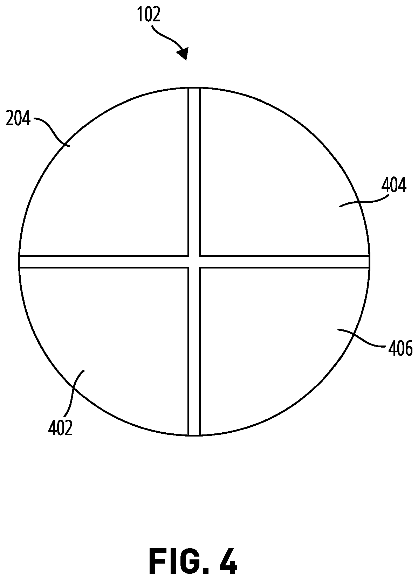

FIG. 1B shows a perspective view of the pod with a vertical plane 110 passing through a vertical axis (Y-axis) of the pod to produce a vertical cross section, shown in FIG. 2. A horizontal plane 112 also passes through a horizontal axis (X-axis) of the pod to produce a horizontal cross section, shown in FIG. 4

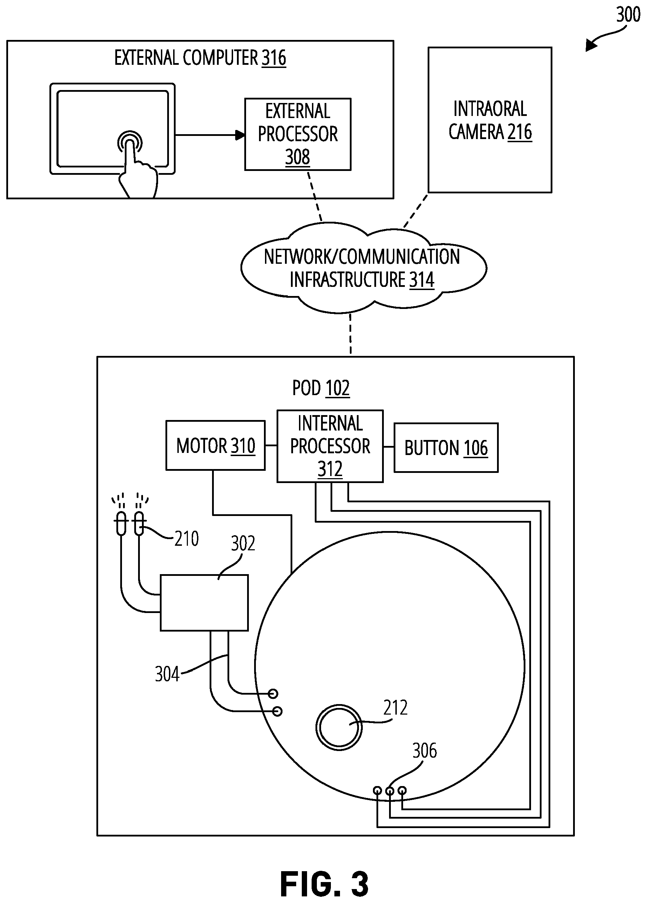

In FIG. 2, the vertical cross sectional view of the pod depicts an illustrative embodiment wherein a receiver 104 has the shape of at least a portion of a distal end 214 of the intraoral camera 216 and is configured for receiving the distal end of the intraoral camera 216. The receiver 104 is also lined with one or more nozzles 210 and/or heating elements 220 to clean, disinfect and/or dry the intraoral camera 216. The nozzles 210 may be configured to apply water, cleaning liquid and/or a disinfecting/sterilizing liquid to at least said distal end 214 in order to clean or disinfect said distal end 214. The liquid, such as water, may be applied under force, such as by the use of a pump 302 (FIG. 3) and ventilator in order to create a spray out of each nozzle 210 to apply pressure when cleaning or disinfecting the distal end 214. The nozzles may also be configured to apply or spray air to the intraoral camera 216 to dry the intraoral camera 216 or at least a distal end 214 end of the intraoral camera 216.

Further a heating/drying plate 208 that lies below the distal end 214 may separately or alternatively apply heat and/or air to dry the distal end 214 of the intraoral camera 216. In particular, the heating elements of the embodiment may be used to dry a lens of the intraoral camera 216. Said heating elements are made of, for example, resistive heating elements which work by converting electrical energy to heat energy. A non-limiting example material suitable for use as a resistive heating element is a Nickel alloy (e.g. NiCr, CuNi) which is a corrosion-resistant material that has sufficient internal electrical resistance, high melting point, and sufficient elevated temperature strength. Due to the use of liquid in the receiver and thus the potential for moisture, a corrosion-resistant resistive heating element may extend the shelf life of the pod 102. In other embodiments, inductive heating elements may be used. This involves heating an electrically conducting object such as a metal by electromagnetic induction, through heat generated in the object by eddy currents. Of course, these examples are not meant to be limiting and other arrangements to produce heat in the pod 102 for heating the distal end 214 can be obtained in light of this specification.

The pod further includes a base portion 222 that includes a cartridge compartment 224 and an engine compartment 226. The cartridge compartment 224 receives one or more cartridges in insertion slots for performing a dental function or workflow, based on, for example, the intraoral camera 216. The dental functions include, for example, maintenance and set-up operations of the intraoral camera that are otherwise performed separately, individually and\or manually by a clinician using external tools and kits. In an illustrative embodiment, the spatial position of cartridges of the cartridge compartment are changeable, relative to the receiver 104 or intraoral camera 216, through a rotation of a motor of the engine compartment 226 as described herein. One or more cartridges may have an entrance 212 for receiving the distal end 214 of the intraoral camera 216 for a dental function. The cross-sectional view of FIG. 2 shows a disinfection cartridge 204 that may hold disinfection liquid and a color calibration cartridge 406 having a color calibration module 202 for calibrating the intraoral camera 216. In another illustrative embodiment (not shown), the spatial position of the receiver 104 or intraoral camera are changeable through a rotation of the intraoral camera or receiver 104 relative to the cartridge compartment 224. The ability of the cartridge compartment 224 to receive a plurality of cartridges allows the ability to change and add functionalities to existing handheld dental devices and to make it versatile for different devices and their features by changing cartridges or developing new cartridges.

In another illustrative embodiment, the performance of the dental functions is automatic and may be controlled by instructions from an internal processor 312 or external processor 308. For example, upon inserting the intraoral camera 216, a processor operates a motor of the engine compartment 226 to rotate the cartridges sequentially to perform one or more dental functions, with each cartridge configured to perform a different function including, but not limited to, 3D calibration, color calibration, cleaning, disinfection and caries detection calibration. In an embodiment, independent cleaning of intraoral camera 216 between the treatments may be achieved. The buttons 106 may also be used by an operator to automatically perform one or more cartridge dependent dental functions, wherein rotation of the cartridge to coincide with the intraoral camera 216 may not be needed if only one dental function is being performed. Each dental function may include a plurality of steps. For example, automatic color calibration includes ensuring that the camera has already been disinfected to remove any substances from the lens, lowering the distal end 214 of the intraoral camera 216 through the entrance 212 into a color calibration module 202 of a color calibration cartridge 406 such that a camera lens 228 of the intraoral camera 216 faces or is mounted on a color calibration set (not shown). Internal processor 312 or external processor 308 then operates the intraoral camera 216 to project images to be reflected and measured in a calibration process. Once complete, an LED (light emitting diode) on the pod 102 or remote software indicates completion and the distal end 214 is moved back up to its previous position. Of course, this is not meant to be limiting and other dental functions can be realized using other types of cartridges configured to perform other dental functions or intraoral camera procedures.

In a further illustrative embodiment, receiver 104 may be reconfigurable, electronically or mechanically to alter the shape of the cavity in order to further lower the distal end 214 of the intraoral camera 216 into an entrance 212 of a calibration module such as the color calibration module 202.

Turning now to FIG. 3, the figure shows a block diagram of a pod system 300 in which illustrative embodiments may be implemented. The pod system 300 includes the pod 102 (a schematic of at least the nozzle structure and data lines of which is shown for illustration purposes). The pod system 300 also includes a button 106, a motor 310 and an internal processor 312 for controlling various operations of the pod. A pump 302 of the pod system 300 operatively connects the nozzle 210 to a disinfectant of liquid supply, such as water (not shown), through one or more hoses 304. The pod system 300 also includes data lines 306 operatively connected to an internal processor 312 or external processor 308 configured to control the performance of dental functions as described herein. Further, the data lines 306 may be configured as extension for the LAN access that is needed for the WLAN access point.

In an illustrative embodiment, the pod 102 is operatively connected to an external computer 316 and/or the intraoral camera 216 through a network/communication infrastructure 314. Network/communication infrastructure 314 is the medium used to provide communications links between various devices, databases, processors and computers connected together within the pod system 300. Network/communication infrastructure 314 may include connections, such as wire, wireless communication links, or fiber optic cables. Data processing systems such as the external computer 316 with external processor 308, intraoral camera 216, internal processor 312, etc. are only examples of certain data processing systems connected to network/communication infrastructure 314 and are not intended to exclude other configurations or roles for these data processing systems. Software applications or software tools may execute on any data processing system in the pod system 300.

Only as an example, and without implying any limitation to such architecture, FIG. 3 depicts certain components that are usable in an example implementation of an embodiment. As another example, an embodiment can be distributed across several data processing systems and a data network as shown, whereas another embodiment can be implemented on a single data processing system within the scope of the illustrative embodiments.

The pod system 300 may be implemented as a number of different types of networks, such as for example, an intranet, a local area network (LAN), or a wide area network (WAN). In a particular example, there is a data communication between the intraoral camera 216 and the pod 102 via a wireless local area network (WLAN) and between the pod and a (W)LAN external computer via LAN. The pod may serve as a WLAN access point for the intraoral camera 216 and as a "hot spot" in dental treatment rooms for dental acquisition units. Herein a clinician may therefore not need a physically connected computer for data processing and data may be sent via LAN to the external computer within a dental office.

Of course, Network/communication infrastructure 314 may also represent a collection of networks and gateways that use the Transmission Control Protocol/Internet Protocol (TCP/IP) and other protocols to communicate with one another. FIG. 3 is intended as an example, and not as an architectural limitation for the different illustrative embodiments.

FIG. 4 shows a horizontal cross-sectional view of the pod 102 with a plurality of cartridges 502 inserted in a cartridge compartment 224. The cartridges include, without limitation, a disinfection cartridge 204, an air-filter cartridge 402, a 3D calibration cartridge 404 and a color calibration cartridge 406.

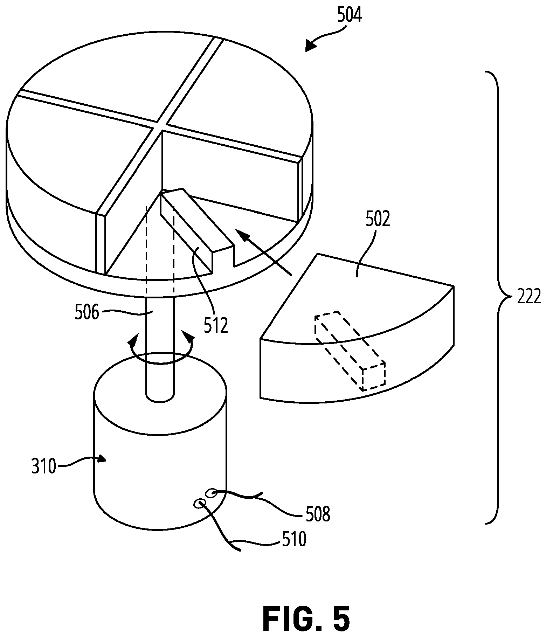

A perspective view of a cartridge assembly 504 is shown in FIG. 5. The cartridge assembly 504 includes, in an illustrative embodiment, one or more cartridges 502, a motor 310 from the engine compartment 226, and a shaft 506 of the motor 310. Insertion guides 512 may guide the insertion and retaining of the one or more cartridges 502 in an illustrative embodiment. The motor 310 also has an encoder line 508 for controlling, responsive to insertion of the intraoral camera 216, the direction of rotation or degree of rotation based on instructions from a processor. The motor also has a power line 510 for supplying power to the motor. A shaft connected from the motor to the cartridge assembly 504 couples rotational movements from the motor to the cartridge assembly 504 such that the cartridges 502 are at defined spatial positions. The processor may also control the movement or reconfiguration of the receiver 104, or movement of the intraoral camera 216, for example by lowering the intraoral camera 216, such that the distal end 214 of the intraoral camera 216, is in alignment with a particular cartridge 502. However, in an illustrative embodiment, the intraoral camera 216 is lowered manually through the entrance 212 by the operator. It is noted that not all cartridges need to have an entrance 212. Further, these examples are not meant to be limiting and other mechanical and electronic movement mechanisms such as other rotary actuators, linear actuators, servos, guide rails, etc., configured to position the cartridges in a predefined alignment with respect to the intraoral camera 216 may be achieved in light of the specification.

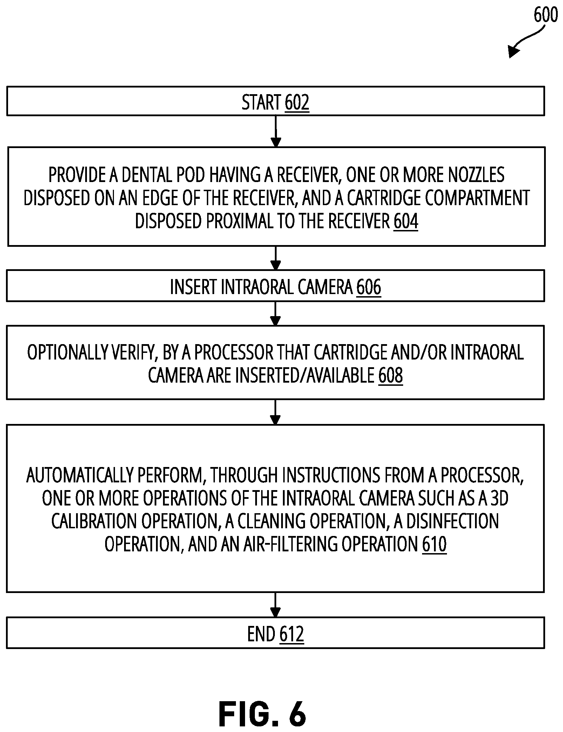

FIG. 6 shows a process 600 in accordance with an illustrative embodiment. The process 600 begins at step 602. In step 604, a pod 102 is provided. The pod 102 has a receiver for at least a distal end of an intraoral camera, one or more nozzles disposed on an edge of the receiver, and a cartridge compartment disposed proximal to the receiver. The intraoral camera is inserted into the pod in step 606. In step 608, a processor optionally verifies or detects that the intraoral camera has been inserted. It may also check that one or more cartridges are available for operations of the intraoral camera. The cartridges are in any shape or form that allows specific instructions of a processor to be carried out on or using the intraoral camera. In step 610, process 600 automatically performs, by the processor, one or more operations of the intraoral camera based on a predefined function of the one or more cartridges. At least one of the one or more operations can be selected from the group consisting of a color calibration operation, a 3D (three-dimensional) calibration operation, a cleaning operation, a disinfection operation, and an air-filtering operation. The process 600 ends at step 612.

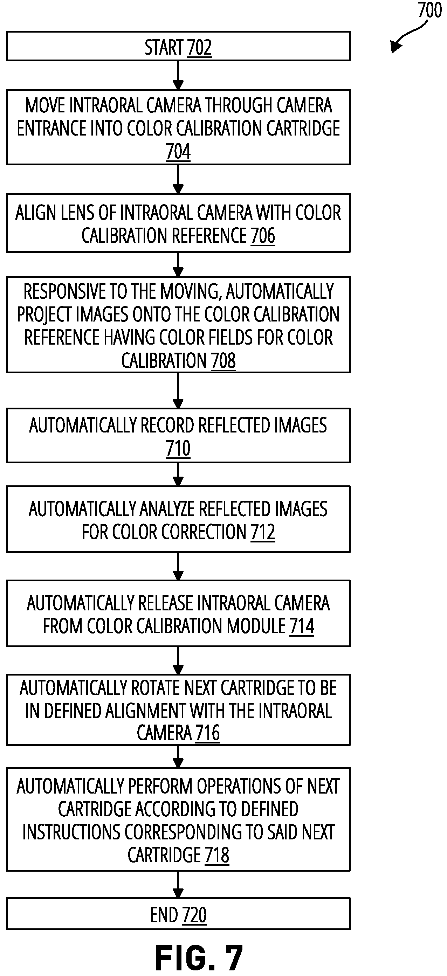

FIG. 7 illustrates another process 700 in accordance with an illustrative embodiment. Process 700 is an example illustration of step 610 of process 600. Process 700 is a color calibration operation and begins at step 702. In step 704, the process 700 moves the intraoral camera through entrance 212 into the color calibration cartridge 406 such that a distal end 214 of the intraoral camera 216 resides in a color calibration module 202 or cavity of the color calibration cartridge 406. In a different operation such as a cleaning, disinfection or heating operation, moving the tip of the intraoral camera 216 through an entrance 212 may not be necessary and the operation may be performed in situ in the receiver 104. In step 706, a lens of intraoral camera is aligned with color calibration reference. The reference may include color fields for color calibration. It may also include white fields and/or gray fields for white balance and/or shading correction. In step 708, responsive to moving the intraoral camera 216 through the entrance 212, the process 700 causes a processor to initiate the automatic projection of images onto the color calibration reference for color calibration. In step 710, the process 700 automatically records reflected images. In step 712, the process 700 automatically analyzes the reflected images for color correction. In step 714, the process 700 automatically releases, by a processor, the intraoral camera from color calibration module. In step 716, the process 700 automatically rotates a next cartridge to be in a defined alignment with the intraoral camera. In step 718, the process 700 automatically performs operations of the next cartridge according to defined instructions corresponding to said next cartridge. The process 700 ends at step 720.

In an example use-case, a clinician, in the morning, takes the charged, cleaned, updated, heated and calibrated intraoral camera 216 out of the pod 102. The clinician uses it on a patient as usual. Data is transferred to a PC and visualized on the monitor, which is connected with the pod 102 via the (W)LAN access point giving it access to the inhouse LAN. After use the intraoral camera 216 is put back into the pod 102. The intraoral camera 216 is subsequently automatically cleaned and charged. It is also automatically heated and calibrated if necessary. The clinician may initiate an additional/separate calibration if needed.

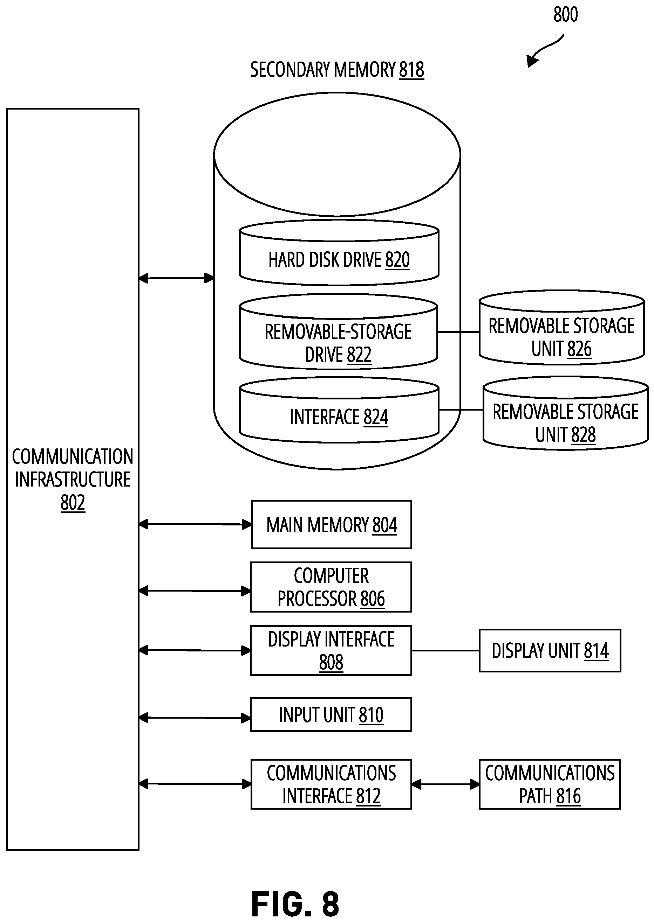

Reference will now be made to FIG. 8, which shows a block diagram of a computer system 800 that may be employed in accordance with at least some of the illustrative embodiments herein. Although various embodiments may be described herein in terms of this exemplary computer system 800, after reading this description, it may become apparent to a person skilled in the relevant art(s) how to implement the pod 102 using other computer systems and/or architectures.

In one example embodiment herein, at least some components of the pod system 300 may form or be included in the computer system 800 of FIG. 8. The computer system 800 includes at least one computer processor 806 which may be, in an exemplary embodiment, internal processor 312 or external processor 308 of the pod system 300. The computer processor 806 may include, for example, a central processing unit (CPU), a multiple processing unit, an application-specific integrated circuit ("ASIC"), a field programmable gate array ("FPGA"), or the like. The computer processor 806 may be connected to a communication infrastructure 802 (e.g. a communications bus, a cross-over bar device, a network). In an illustrative embodiment herein, the computer processor 806 includes a CPU that that controls operations of the pod 102, including the automatic cleaning and disinfection processes. For example, the computer processor 806 may be configured to control the timing and sequence of said operations.

The display interface 808 (or other output interface) forwards text, video graphics, and other data from the communication infrastructure 802 (or from a frame buffer (not shown)) for display on display unit 814. For example, the display interface 808 may include a video card with a graphics processing unit or may provide an operator with an interface for controlling the thermoforming apparatus.

The computer system 800 may also include an input unit 810 that may be used, along with the display unit 814 by an operator of the computer system 800 to send information to the computer processor 806. The input unit 810 may include a keyboard, buttons 106 and/or touchscreen monitor on or outside of the pod 102 for sending instructions for execution to a processor. In one example, the display unit 814, the input unit 810, and the computer processor 806 may collectively form a user interface.

One or more steps of operating the pod 102 may be stored as instructions on a non-transitory storage device in the form of computer-readable program instructions. To execute a procedure, the computer processor 806 loads the appropriate instructions, as stored on storage device, into memory and then executes the loaded instructions.

The computer system 800 may further comprise a main memory 804, which may be a random-access memory ("RAM"), and also may include a secondary memory 818. The secondary memory 818 may include, for example, a hard disk drive 820 and/or a removable-storage drive 822 (e.g., a floppy disk drive, a magnetic tape drive, an optical disk drive, a flash memory drive, and the like). The removable-storage drive 822 reads from and/or writes to a removable storage unit 826 in a well-known manner. The removable storage unit 826 may be, for example, a floppy disk, a magnetic tape, an optical disk, a flash memory device, and the like, which may be written to and read from by the removable-storage drive 822. The removable storage unit 826 may include a non-transitory computer-readable storage medium storing computer-executable software instruction and/or data.

In further illustrative embodiments, the secondary memory 818 may include other computer-readable media storing computer-executable programs or other instructions to be loaded into the computer system 800. Such devices may include removable storage unit 828 and an interface 824 (e.g., a program cartridge and a cartridge interface); a removable memory chip (e.g., an erasable programmable read-only memory ("EPROM") or a programmable read-only memory ("PROM")) and an associated memory socket; and other removable storage units 828 and interfaces 824 that allow software and data to be transferred from the removable storage unit 828 to other parts of the computer system 800.

The computer system 800 may also include a communications interface 812 that enables software and data to be transferred between the computer system 800 and external devices. Such an interface may include a modem, a network interface (e.g., an Ethernet card or an IEEE 602.11 wireless LAN interface), a communications port (e.g., a Universal Serial Bus ("USB") port or a FireWire^{®} port), a Personal Computer Memory Card International Association ("PCMCIA") interface, Bluetooth^{®}, and the like. Software and data transferred via the communications interface 812 may be in the form of signals, which may be electronic, electromagnetic, optical or another type of signal that may be capable of being transmitted and/or received by the communications interface 812. Signals may be provided to the communications interface 812 via a communications path 816 (e.g., a channel). The communications path 816 carries signals and may be implemented using wire or cable, fiber optics, a telephone line, a cellular link, a radiofrequency ("RF") link, or the like. The communications interface 812 may be used to transfer software or data or other information between the computer system 800 and a remote server or cloud-based storage (not shown).

One or more computer programs or computer control logic may be stored in the main memory 804 and/or the secondary memory 818. The computer programs may also be received via the communications interface 812. The computer programs include computer-executable instructions which, when executed by the computer processor 806, cause the computer system 800 to perform the methods as described hereinafter. Accordingly, the computer programs may control the computer system 800 and other components of the pod 102.

In another embodiment, the software may be stored in a non-transitory computer-readable storage medium and loaded into the main memory 804 and/or the secondary memory 818 of the using the removable-storage drive 822, hard disk drive 820, and/or the communications interface 812. Control logic (software), when executed by the computer processor 806, causes the computer system 800, and more generally the pod system 300, to perform the some or all of the methods described herein.

Lastly, in another example embodiment hardware components such as ASICs, FPGAs, and the like, may be used to carry out the functionality described herein. Implementation of such a hardware arrangement so as to perform the functions described herein will be apparent to persons skilled in the relevant art(s) in view of this description.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A dental pod (102) comprising:
a receiver (104) in a form of a cavity, adapted to receive a distal end (214) of a dental handheld device (216);
one or more nozzles disposed on a side of the receiver, the one or more nozzles configured to clean or disinfect the distal end of the dental handheld device;
**characterised in that** it further comprises
a cartridge compartment (224) disposed proximal to the receiver, the cartridge compartment including more than one insertion slot, wherein the insertion slots are for cartridges (204,402,404,406), and wherein the cartridges being configured to support operations of the handheld device, wherein each cartridge is one of a color calibration cartridge (406), a 3D calibration cartridge (404), a disinfection cartridge (204), a dental caries cartridge and an air-filter cartridge (402); and
a motor (310) adapted to turn the one or more cartridges to a predefined spatial position relative to the receiver to perform said operations in the dental handheld device; and
a processor (31, adapted to automatically perform operations of the dental handheld device based on a predefined function of the cartridges, at least one of the operations being selected from the group consisting of a color calibration operation, a 3D calibration operation, a cleaning operation, a disinfection operation, caries detection calibration, and an air-filtering operation.

2. The dental pod of claim 1, wherein the dental handheld device is an intraoral camera, a dental caries detector or an apex locator.

3. The dental pod of claim 1, wherein the pod is configured to alter a shape of the receiver to move the distal end of the dental handheld device into or out of an entrance of the one or more cartridges.

4. The dental pod of claim 1, wherein the dental pod includes a heater disposed along the side of the receiver or proximal to the receiver to heat at least the distal end of the dental handheld device after a cleaning operation.

5. The dental pod of claim 1, wherein the one or more nozzles is operatively coupled to a pump for delivering fluids to the distal end of the dental handheld device.

6. The dental pod of claim 5, wherein the fluids include at least one of fluids selected from the group consisting of spray water, spray air, a cleaning liquid and a disinfectant.

7. The dental pod of claim 1, wherein the cartridge compartment is disposed below the receiver.

8. The dental pod of claim 7, wherein an engine compartment is disposed below the cartridge compartment.

9. The dental pod of claim 1, wherein the pod is configured to communicate wirelessly with the dental handheld device.

10. The dental pod of claim 1, wherein one or more insertion guides guide the insertion and retaining of the one or more cartridges in the cartridge compartment.

11. The dental pod of claim 1, wherein an internal processor of the pod or an external processor is configured to control said operations of the dental handheld device.

12. The dental pod of claim 1, wherein the dental pod includes at least one of a WLAN access point, an LAN slot and a plug for charging a battery.

13. The dental pod of claim 1, wherein the receiver is shaped to allow manual movement of the distal end of the dental handheld device into or out of an entrance of the one or more cartridges.

14. A computer-assisted method of operating a dental pod as defined in any one of claims 1-13, comprising the step of performing, by a processor (308;312), one or more operations of the dental handheld device based on a predefined function of the one or more cartridges, at least one of the one or more operations being selected from the group consisting of a color calibration operation, a 3D(three-dimensional) calibration operation, a cleaning operation, a disinfection operation, caries detection calibration, and an air-filtering operation.

15. The method of claim 14, wherein the automatically performing step includes a maintenance step of the dental handheld device, an automatic set-up step of the dental handheld device or an automatic cleaning step of the dental handheld device.

16. The method of claim 14, further comprising:
operating a motor to turn at least one of the one or more cartridges to a predefined spatial position that is in alignment with the distal end of the dental handheld device in order to perform said one or more operations of the dental handheld device.

17. The method of claim 14, further comprising:
verifying that a distal end of the dental handheld device has been received by the receiver;
verifying that one or more cartridges are available in the cartridge compartment, the one or more cartridges being configured to support one or more operations of the dental handheld device.

18. The method of claim 14, further comprising transferring data from the dental handheld device to a computer via a network of the pod.

19. The method of claim 14, wherein the dental handheld device is cleaned, heated and calibrated automatically.

20. The method of claim 14, wherein the dental handheld device is an intraoral camera, a dental caries detector or an apex locator

## Patentansprüche

1. Dental-Pod (102), umfassend:
eine Aufnahme (104) in einer Form eines Hohlraums, die dazu angepasst ist, ein distales Ende (214) eines zahnärztlichen Handgeräts (216) aufzunehmen;
eine oder mehrere Düsen, die an einer Seite der Aufnahme angeordnet sind, wobei die eine oder die mehreren Düsen dazu konfiguriert sind, das distale Ende des zahnärztlichen Handgeräts zu reinigen oder zu desinfizieren;
**dadurch gekennzeichnet, dass** er ferner Folgendes umfasst
ein Patronenfach (224), das proximal zu der Aufnahme angeordnet ist, wobei das Patronenfach mehr als einen Einführschlitz beinhaltet, wobei die Einführschlitze für Patronen (204,402,404,406) sind und wobei die Patronen dazu konfiguriert sind, Vorgänge des Handgeräts zu unterstützen, wobei jede Patrone eine von einer Farbkalibrierungspatrone (406), einer 3D-Kalibrierungspatrone (404), einer Desinfektionspatrone (204), einer Zahnkariespatrone und einer Luftfilterpatrone (402) ist; und
einen Motor (310), der dazu angepasst ist, die eine oder mehreren Patronen in eine vordefinierte räumliche Position in Bezug auf die Aufnahme zu drehen, um die Vorgänge in dem zahnärztlichen Handgerät durchzuführen; und
einen Prozessor (3), der dazu angepasst ist, automatisch Vorgänge des zahnärztlichen Handgeräts basierend auf einer vordefinierten Funktion der Patronen durchzuführen, wobei mindestens einer der Vorgänge aus der Gruppe ausgewählt ist, die aus einem Farbkalibrierungsvorgang, einem 3D-Kalibrierungsvorgang, einem Reinigungsvorgang, einem Desinfektionsvorgang, einer Karieserkennungskalibrierung und einem Luftfiltrationsvorgang besteht.

2. Dental-Pod nach Anspruch 1, wobei das zahnärztliche Handgerät eine intraorale Kamera, ein Zahnkariesdetektor oder ein Spitzenlokalisator ist.

3. Dental-Pod nach Anspruch 1, wobei der Pod dazu konfiguriert ist, eine Form der Aufnahme zu ändern, um das distale Ende des zahnärztlichen Handgeräts in oder aus einem Eingang der einen oder mehreren Patronen zu bewegen.

4. Dental-Pod nach Anspruch 1, wobei der Dental-Pod einen Heizkörper beinhaltet, der entlang der Seite der Aufnahme oder proximal zu der Aufnahme angeordnet ist, um zumindest das distale Ende des zahnärztlichen Handgeräts nach einem Reinigungsvorgang zu erwärmen.

5. Dental-Pod nach Anspruch 1, wobei die eine oder mehreren Düsen betriebsmäßig an eine Pumpe gekoppelt sind, um Fluide an das distale Ende des zahnärztlichen Handgeräts zu liefern.

6. Dental-Pod nach Anspruch 5, wobei die Fluide mindestens eines von Fluiden beinhalten, die aus der Gruppe ausgewählt sind, die aus Sprühwasser, Sprühluft, einer Reinigungsflüssigkeit und einem Desinfektionsmittel besteht.

7. Dental-Pod nach Anspruch 1, wobei das Patronenfach unter der Aufnahme angeordnet ist.

8. Dental-Pod nach Anspruch 7, wobei ein Motorfach unter dem Patronenfach angeordnet ist.

9. Dental-Pod nach Anspruch 1, wobei der Pod dazu konfiguriert ist, drahtlos mit dem zahnärztlichen Handgerät zu kommunizieren.

10. Dental-Pod nach Anspruch 1, wobei eine oder mehrere Einführführungen das Einführen und Halten der einen oder mehreren Patronen in dem Patronenfach führen.

11. Dental-Pod nach Anspruch 1, wobei ein interner Prozessor des Pods oder ein externer Prozessor dazu konfiguriert ist, die Vorgänge des zahnärztlichen Handgeräts zu steuern.

12. Dental-Pod nach Anspruch 1, wobei der Dental-Pod mindestens einen von einem WLAN-Zugangspunkt, einem LAN-Steckplatz und einem Stecker zum Laden einer Batterie beinhaltet.

13. Dental-Pod nach Anspruch 1, wobei die Aufnahme so geformt ist, dass sie eine manuelle Bewegung des distalen Endes des zahnärztlichen Handgeräts in oder aus einem Eingang der einen oder mehreren Patronen ermöglicht.

14. Computergestütztes Verfahren zum Betreiben eines Dental-Pods wie in einem der Ansprüche 1-13 definiert,
umfassend den Schritt des Durchführens eines oder mehrerer Vorgänge des zahnärztlichen Handgeräts durch einen Prozessor (308;312) basierend auf einer vordefinierten Funktion der einen oder mehreren Patronen, wobei mindestens einer des einen oder der mehreren Vorgänge aus der Gruppe ausgewählt ist, die aus einem Farbkalibrierungsvorgang, einem 3D(dreidimensionalen)-Kalibrierungsvorgang, einem Reinigungsvorgang, einem Desinfektionsvorgang, einer Karieserkennungskalibrierung und einem Luftfiltrationsvorgang besteht.

15. Verfahren nach Anspruch 14, wobei der Schritt des automatischen Durchführens einen Wartungsschritt des zahnärztlichen Handgeräts, einen Schritt des automatischen Einrichtens des zahnärztlichen Handgeräts oder einen Schritt des automatischen Reinigens des zahnärztlichen Handgeräts beinhaltet.

16. Verfahren nach Anspruch 14, ferner umfassend:
Betreiben eines Motors, um mindestens eine der einen oder mehreren Patronen in eine vordefinierte räumliche Position zu drehen, die mit dem distalen Ende des zahnärztlichen Handgeräts ausgerichtet ist, um den einen oder die mehreren Vorgänge des zahnärztlichen Handgeräts durchzuführen.

17. Verfahren nach Anspruch 14, ferner umfassend:
Überprüfen, dass ein distales Ende des zahnärztlichen Handgeräts von der Aufnahme aufgenommen worden ist;
Überprüfen, dass eine oder mehrere Patronen in dem Patronenfach verfügbar sind, wobei die eine oder mehreren Patronen dazu konfiguriert sind, einen oder mehrere Vorgänge des zahnärztlichen Handgeräts zu unterstützen.

18. Verfahren nach Anspruch 14, ferner umfassend das Übertragen von Daten von dem zahnärztlichen Handgerät über ein Netzwerk des Pods an einen Computer.

19. Verfahren nach Anspruch 14, wobei das zahnärztliche Handgerät automatisch gereinigt, erwärmt und kalibriert wird.

20. Verfahren nach Anspruch 14, wobei das zahnärztliche Handgerät eine intraorale Kamera, ein Zahnkariesdetektor oder ein Spitzenlokalisator ist.

## Revendications

1. Nacelle dentaire (102) comprenant :
un récepteur (104) sous la forme d'une cavité, adapté pour recevoir une extrémité distale (214) d'un dispositif portatif dentaire (216) ;
une ou plusieurs buses disposées sur un côté du récepteur, lesdites une ou plusieurs buses étant conçues pour nettoyer ou désinfecter l'extrémité distale du dispositif portatif dentaire ;
**caractérisée en ce qu'**elle comprend en outre
un compartiment de cartouches (224) disposé à proximité du récepteur, le compartiment de cartouches comprenant plus d'une fente d'insertion, lesdites fentes d'insertion étant destinées aux cartouches (204,402,404,406), et lesdites cartouches étant conçues pour supporter les opérations du dispositif portatif, chaque cartouche étant une cartouche parmi une cartouche d'étalonnage de couleur (406), une cartouche d'étalonnage 3D (404), une cartouche de désinfection (204), une cartouche de caries dentaires et une cartouche de filtre à air (402) ; et
un moteur (310) adapté pour faire tourner lesdites une ou plusieurs cartouches vers une position spatiale prédéfinie par rapport au récepteur de manière à effectuer lesdites opérations dans le dispositif portatif dentaire ; et
un processeur (3), adapté pour effectuer automatiquement des opérations du dispositif portatif dentaire sur la base d'une fonction prédéfinie des cartouches, au moins l'une des opérations étant choisie dans le groupe constitué par une opération d'étalonnage de couleur, une opération d'étalonnage 3D, une opération de nettoyage, une opération de désinfection, un étalonnage de détection de caries et une opération de filtration d'air.

2. Nacelle dentaire selon la revendication 1, ledit dispositif portatif dentaire étant une caméra intra-buccale, un détecteur de caries dentaires ou un localisateur d'apex.

3. Nacelle dentaire selon la revendication 1, ladite nacelle étant conçue pour modifier la forme du récepteur de manière à déplacer l'extrémité distale du dispositif portatif dentaire dans ou hors d'une entrée desdites une ou plusieurs cartouches.

4. Nacelle dentaire selon la revendication 1, ladite nacelle dentaire comprenant un élément chauffant disposé le long du côté du récepteur ou à proximité du récepteur de manière à chauffer au moins l'extrémité distale du dispositif portatif dentaire après une opération de nettoyage.

5. Nacelle dentaire selon la revendication 1, lesdites une ou plusieurs buses étant couplées de manière fonctionnelle à une pompe de manière à délivrer des fluides au niveau de l'extrémité distale du dispositif portatif dentaire.

6. Nacelle dentaire selon la revendication 5, lesdits fluides comprenant au moins l'un des fluides choisis dans le groupe constitué par l'eau de pulvérisation, l'air de pulvérisation, un liquide de nettoyage et un désinfectant.

7. Nacelle dentaire selon la revendication 1, ledit compartiment de cartouches étant disposé au-dessous du récepteur.

8. Nacelle dentaire selon la revendication 7, un compartiment moteur étant disposé en dessous du compartiment de cartouches.

9. Nacelle dentaire selon la revendication 1, ladite nacelle étant conçue pour communiquer sans fil avec le dispositif portatif dentaire.

10. Nacelle dentaire selon la revendication 1, un ou plusieurs guides d'insertion guidant l'insertion et la retenue desdites une ou plusieurs cartouches dans le compartiment de cartouches.

11. Nacelle dentaire selon la revendication 1, un processeur interne de la nacelle ou un processeur externe étant conçu pour commander lesdites opérations du dispositif portatif dentaire.

12. Nacelle dentaire selon la revendication 1, ladite nacelle dentaire comprenant au moins un point d'accès WLAN, une fente LAN et une fiche pour charger une batterie.

13. Nacelle dentaire selon la revendication 1, ledit récepteur étant conçu pour permettre un déplacement manuel de l'extrémité distale du dispositif portatif dentaire dans ou hors d'une entrée desdites une ou plusieurs cartouches.

14. Procédé assisté par ordinateur de fonctionnement d'une nacelle dentaire selon l'une quelconque des revendications 1 à 13, comprenant l'étape de réalisation, par un processeur (308;312), d'une ou plusieurs opérations du dispositif portatif dentaire sur la base d'une fonction prédéfinie desdites une ou plusieurs cartouches, au moins l'une desdites une ou plusieurs opérations étant choisie dans le groupe constitué par une opération d'étalonnage de couleur, une opération d'étalonnage 3D (tridimensionnel), une opération de nettoyage, une opération de désinfection, un étalonnage de détection de caries et une opération de filtration d'air.

15. Procédé selon la revendication 14, ladite étape d'exécution automatique comprenant une étape de maintenance du dispositif portatif dentaire, une étape de réglage automatique du dispositif portatif dentaire ou une étape de nettoyage automatique du dispositif portatif dentaire.

16. Procédé selon la revendication 14, comprenant en outre :
le fonctionnement d'un moteur de manière à faire tourner au moins l'une desdites une ou plusieurs cartouches vers une position spatiale prédéfinie qui est alignée avec l'extrémité distale du dispositif portatif dentaire de manière à effectuer lesdites une ou plusieurs opérations du dispositif portatif dentaire.

17. Procédé selon la revendication 14, comprenant en outre :
la vérification qu'une extrémité distale du dispositif portatif dentaire a été reçue par le récepteur ;
la vérification qu'une ou plusieurs cartouches sont disponibles dans le compartiment de cartouches, lesdites une ou plusieurs cartouches étant conçues pour supporter une ou plusieurs opérations du dispositif portatif dentaire.

18. Procédé selon la revendication 14, comprenant en outre le transfert de données du dispositif dentaire portatif à un ordinateur par l'intermédiaire d'un réseau de la nacelle.

19. Procédé selon la revendication 14, ledit dispositif dentaire portatif étant nettoyé, chauffé et étalonné automatiquement.

20. Procédé selon la revendication 14, ledit dispositif portatif dentaire étant une caméra intra-buccale, un détecteur de caries dentaires ou un localisateur d'apex.
